# EUROPEAN PATENT APPLICATION

(11) **EP 1 300 786 A1**
(43) Date of publication of application: **09.04.2003**
(21) Application number: 01934453.0
(22) Date of filing: 31.05.2001
(51) Int. Cl.: G06F 17/60

(54) **MEDICAL EXAMINATION NETWORK SYSTEM**

(30) Priority: 31.05.2000 JP 2000162012; 07.06.2000 JP 2000170126; 30.06.2000 JP 2000198328
(71) Applicant: MATSUSHITA ELECTRIC INDUSTRIAL CO., LTD., Kadoma-shi, Osaka 571-8501 (JP)
(72) Inventor: NAGAMOTO, Shunichi, Nara-shi, Nara 631-0803 (JP); NOMURA, Hiroyoshi, Kizu-cho, Souraku-gun, Kyoto 619-0224 (JP); YASUI, Toshihiko, Nara-shi, Nara 631-0074 (JP); KANAZAWA, Kiyoshi, Katano-shi, Osaka 576-0051 (JP); IMAI, Hirohisa, Nara-shi, Nara 630-8444 (JP); YAMASHITA, Kunihiko, Ikoma-shi, Nara 630-0122 (JP); TANIE, Katsunori, Moriguchi-shi, Osaka 570-0008 (JP); KOBAYASHI, Tetsu, Nara-shi, Nara 630-8423 (JP)
(74) Representative: Eisenführ, Speiser & Partner
(86) International application number: JP0104592
(87) International publication number: WO01093140

(57) **Abstract**

A medical checkup network system includes a patient terminal (1) for measuring the biodata of each patient including a blood pressure and a body temperature, a doctor terminal (2) for allowing the medical staff to view the biodata of each patient for diagnosis and enter a medical support data including a schedule data and an advice data, and a center server (3) for storing the data received from the those terminals. They are connected to each other over a communication network (4) such as the Internet. In the system, the biodata of each patient stored in the center server (3) can be registered and viewed by any of the patient terminal (1), the doctor terminal (2), the patient and the medical staff, those having been registered in the center server. The system can thus protect the privacy of each patient about the biodata and permit the biodata of each patient to be shared by two or more members of the medical staff.

## Description

### Technical Field

The present invention relates to a medical checkup network system for exchanging medical support data and patient biodata between patients and medical staff including doctors and nurses. Particularly, the invention relates to a technology of developing a medical checkup network system for transmitting over a communication network and storing in a center server a variety of confidential data such as medical support data and patient biodata to carry out a bidirectional medical data exchange.

### Background Art

As the social demand for limiting the soaring medical costs is increased, the medical treatment and care of patients at home is now focused. Also, in the aging society, it becomes strongly desired to routinely check the biodata of each personal for staying healthy and enjoying the elderly life or a quality of life (QOL). For realizing the medical treatment and care at home, medical checkup network systems have been developed.

Such a medical checkup network system is arranged in which a patient terminal is provided at each patient's home for measuring biodata of the patient, transmitting them to a doctor terminal over a telephone line, and receiving several medical services from a team of medical staff over the telephone line. This system allows the patient who has to receive the medical treatment and care at regular intervals to reduce the frequency of visits to clinics or hospitals or the number of requests of hose calls. Also, using an advanced, recently introduced Internet technology, the biodata of each patient received and stored in a web server can be accessed over the Internet from any location to provide a diagnosis readily and accurately.

One of the conventional medical checkup network systems is disclosed in Japanese Patent Laid-open Publication No. 11-85891.

The disclosed system comprises a health care apparatus and a client terminal. The health care apparatus has a web server functions for measuring biodata of each patient or client and storing their data together with relevant medical data in the HTML format. The client terminal has a web browser function for browsing those data over the Internet using the HTTP protocol.

The system allows each patient to operate its health care apparatus (patient terminal) for measuring its biodata at home without visiting a distant clinic or hospital. On the other hand, the system permits doctors or nurses to access the biodata of a desired patient measured and stored in the health care apparatus from the client (doctor) terminal located at the clinic, hospital or their home. As a result, a diagnosis of the patient can readily be made and its treatment and care will be provided quickly.

However, the above system has the following drawback.

As the biodata is stored in the web server of its health care apparatus, and can easily be accessed from any unknown client terminal over the Internet. Therefore, a security scheme is provided. One of effective countermeasures is to limit client terminals which are accessible. Such a security scheme is implemented by a fire-wall device. The fire-wall device has to be installed in each health care apparatus which acts as a server, hence increasing the overall cost of the system. Also, the system will be complex in the management or control.

Other similar medical checkup network systems are disclosed in Japanese Patent No.1986420 and Japanese Patent Laid-open Publication No. 10-328147.

Each portable medical device (patient terminal) in the other systems comprises a central processing unit (CPU) for controlling the operation of each component and exchanging data between the components, a memory for storing the data, an interface for communicating with various sensors to measure the biodata, an entry section comprising a touch panel or keypad through which the patient entries data or selects items, a display comprising a LCD screen for displaying the input data and measured data, and a transceiver for transmitting and receiving data from a host computer (the doctor terminal or a center server) over the public circuit.

In such a configuration, a measuring sensor is selected by the patient according to a display on the display, and then the measured data is picked up and stored in the memory by the CPU. After the measurement is completed, the measurement data is selectively picked up from the memory and transmitted by the CPU from the transmitter to the host computer over the public circuit.

The above system, however, has also the following drawback. Although functions to be required on the portable medical device are varied depending on the conditions of patients, patient data and a method of setting the physical sensor to be used are not considered in the portable medical device. For example, if the portable medical device is designed to be operable to all the diagnoses of each patient, it provides a great number of functions regardless of the patient's individual diagnosis, and thus the arrangement becomes too complicate to operate. Also, in case that a plurality of patients share one portable medical device, it needs to manage information of measuring sensors and storage of the measured data for each patient. This may hardly be covered by the portable medical devices of the conventional systems.

Further medical checkup network systems are disclosed in Japanese Patent Application No. 4-290194 and Japanese Patent Laid-open Publication No. 7-116128.

Those systems are arranged not only for measuring and transferring the biodata of each patient to a medical facility but also for controlling schedules of measuring the biodata at equal intervals of a time and carrying out an action of treatment and care in routine. The schedule data consists of a pair of the time data and the medical activity data including the measurement and the dosage of medicines. The systems allow the schedule data to be directly entered to the (patient) measuring terminal for the biodata by each patient or determined by a doctor or nurse over the communication network. When the schedule data is entered directly by the patient, it may be stored and controlled in the measuring terminal of the patient. When the schedule data is determined by the doctor or nurse, it is stored and controlled in the terminal of the doctor or nurse and can be downloaded to the measuring terminal of the patient if desired. In any case, when the time listed in the schedule is up, its related instruction is notified to the patient by buzzer sounds or the like.

The above systems also have a drawback.

In case that the schedule data is entered to the measuring terminal and controlled directly by the patient, it may be found defective due to entry error. Such entry error in the schedule data can hardly be specified by the doctor terminal. Also, the dosage of medicines and the measurement of the biodata of a patient may be instructed and supervised by not only a single doctor but also a group of medical staff including doctors, nurses, and attorneys. When the schedule data of a patient is registered and stored at one single doctor terminal, it may hardly be shared by two or more members of the medical staff such as doctors, nurses, and care people because the patient information is stored in the doctor terminal.

The measurement terminal for measuring the biodata may also be shared by two or more patients but not always located close to the patient to be examined. When the reception of the schedule data is notified with the use of buzzer sounds, it may hardly be perceived by the patient. Also, the buzzer sound from the terminal fails to tell the assignment of the schedule data to the patient.

### Disclosure of Invention

It is an object of the present invention to provide a medical checkup network system which comprises a patient terminal, a doctor terminal and a center server. The patient terminal measures the biodata of each patient. The doctor terminal displays the biodata measured by the patient terminal to carry out a medical treatment, and enters a medical support data for the patient therein. The center server stores the biodata measured by the patient terminal and provides the doctor terminal with the biodata or its modification. Further the server stores the medical support data entered by the doctor terminal, and provides the patient terminal with the medical support data or its modification. Those are connected to each other over a communication network.

The medical checkup network system may include a security manageing section for protecting confidentiality which permits the biodata and the medical support data stored in the center server to be accessed only by the patient, the patient terminal, the medical staff, and the doctor terminal registered in the center server.

The system of the present invention has at least one procedure of registering the patient terminal data in the center server selected from a procedure of entering or inputting identification number identifying the patient terminal, a procedure of entering a patient name, a procedure of entering identification code corresponding to the patient name, a procedure of entering at least one biodata item of the patient to be measured corresponding to the patient name, and a procedure of entering an instrument name of a health sensor for measuring the biodata.

In the patient terminal provided in the patient's home, the system has also a procedure of connecting to the center server over the communication network, a procedure of receiving from the center server patient terminal data which includes the name of the patient corresponding to the identification number of the patient terminal, the identification code corresponding to the patient name, the biodata item of the patient to be measured, the instrument name of the sensor for measuring the biodata, and the data for controlling the sensor.

The doctor terminal of the system also has the same procedures, except of the requirements for the sensor.

In the system, the actions of the patient terminal and the doctor terminal can be monitored and controlled by a designated system manager operating the administrator terminal. The registration or removal of the data can properly be carried out by a strictly controlled manner and the security of the system will be kept monistically at higher level. Also, the patient terminal permits two or more patients to be assigned for use with their biodata registered.

The system of the present invention permits the schedule data which determines the medical activities of each patient to be managed by the center server so that relevant data can be generated, modified, and monitored by two or more of the doctor terminals. In addition, the schedule data as well as the name of the patient can be displayed in the form of messages, images and sounds. The patient terminal may include an entry device for inputting the result of medical activities of the patient determined by the schedule data.

According to the arrangement described above, the biodata and the schedule data of each patient can be monitored and registered by a plurality of doctor terminals while the result of medical activities of the patient performed according to the schedule data can be monitored by the same. The schedule data for health care may be received by not only the patient terminal for measuring the biodata but also another applicable device such as a mobile phone or a home computer when employing an advanced communicating means such as an electronic mail (e-mail). This allows the patient terminal to be shared by a plurality of patients.

In a first aspect of the invention, a medical checkup network system comprises a patient terminal for measuring a predetermined biodata of each patient such as a blood pressure or a body temperature, a center server for storing the biodata measured by the patient terminal, and a doctor terminal for displaying the biodata stored in the center server to conduct a diagnosis. The patient terminal, the doctor terminal, and the center server are connected with each other over a communication network. This allows the biodata of the patient to be displayed or monitored by the medical staff anywhere, hence ensuring a quick and efficient action of the medical treatment at a remote place. Also, the patient can daily check his biodata and get high satisfaction with remote care by the medical staff. As the biodata of each patient is checked from different directions by a plurality of the medical staff, a resultant medical treatment will be enhanced.

In a second aspect of the invention, a medical checkup network system comprises a doctor terminal for entering a predetermined medical support data such as an advice data or a schedule data to the patient, a center server for storing the medical support data entered by the doctor terminal, and a patient terminal for displaying the medical support data received from the center server. The patient terminal, the doctor terminal, and the center server are connected with each other over a communication network. This allows the medical support data to be readily transferred from the medical staff to the patient, thus increasing the level of diagnosis.

In the network system, the center server may have an authorizing section for permitting the patient, the patient terminal, the medical staff, or the doctor terminal registered in the center server to enter a data or access the data stored in the center server. This allows confidential data such as the biodata of the patient stored in the center server to be accessed for registration or modification by the patient terminal, the doctor terminal, the patient, and the medical staff which are registered in the center server. Accordingly, the system will be improved in security management.

In the network system, the center server may have an administrator terminal function for registering the user of the system and inputting the various medical data in the center server. This allows the setting, action, and state of each of the patient terminal and the doctor terminal to be monitored by the administrator terminal, hence increasing the efficiency of management of the center server.

In the network system, the center server may store at least one software content of the patient terminal, the doctor terminal, or the administrator terminal. Each of the terminals may download the software content from the center server. As a result, the content or function of the terminals can easily be modified by the center server thus improving the efficiency of the overall operation.

In the network system, any of the patient terminal, the doctor terminal, and the administrator terminal may include a section for downloading an update version of the software content for version up. This allows the patient terminal to compare the current version of the software content with an update version data stored in the center server and when they are different, systematically downloads an update version of the software content. Consequently, the software of the latest version can always be used.

In the network system, the doctor terminal may include a section for checking whether or not the advice data for the patient is received by the patient terminal. If the advice data dispatched from the medical staff to the patient is not timely read by the patient, the data may be useless. The above arrangement permits the medical staff to know whether or not the advice data is duly received by the patient terminal, thus ensuring a favorable degree of aftercare.

In the network system, the administrator terminal may have a section for registering an access right of the patient, the patient terminal, the doctor, or the doctor terminal to access the center server. This allows the registration or removal of data to be carried out by a specific system manager leading a strictly controlled manner. As a result, the system will be improved in the monistical security control.

In the network system, the administrator terminal may have a section for entering a patient terminal data which is a data about the patient terminal to be used by the patient. This allows each patient terminal or each of the patients at their patient terminals to be remotely controlled from the administrator terminal. As a result, the action of the medical checkup network system can be controlled monistically at high efficiency.

In the network system, the administrator terminal may perform at one of procedures comprising a procedure of entering the patient terminal data which are a procedure of entering the identification number of the patient terminal, a procedure of entering the name of the patient corresponding to the identification number, a procedure of entering the identification code corresponding to the patient name, a procedure of entering at least one or more desired biodata of the patient to be measured, and a procedure of entering the name of each sensor instrument used for measuring the biodata.

In the network system, the doctor terminal may have a biodata threshold setting section for setting a threshold of the biodata of each patient. The center server receives and examines the threshold determined by the doctor terminal and when the level of the biodata of the patient measured by the patient terminal is within a predetermined range determined by the threshold, notifies the doctor terminal of the fact. This allows the care level of the patient to be determined by the doctor or medical staff depending on the biodata of the patient and in case of emergency, an optimum treatment will be expected.

In the network system, the doctor terminal may have a sensor sensitivity setting section for determining the level of sensitivity to a sensor signal of the patient terminal. This allows the level of the biodata which may be different between the patients to be modified by the doctor terminal for having an optimum accurate measurement. In particular, this feature is effective to the stethoscope signal or electrocardiograph signal which exhibits a large difference between the patients.

In the network system, the patient terminal systematically may start communicating with the center server upon being energized. Before the patient operates the patient terminal, an initial connection process is commenced including automatic updating of the software content, reception of the medical support data, and transmission of not-dispatched biodata. This allows the patient to operate the patient terminal of the latest condition.

In the network system, the patient terminal may have a communicating section for transmitting at least one measurement of the biodata of the patient to the center server. The center server may have a database for storing the biodata received from the patient terminal. The doctor terminal may have a biodata displaying section for communicating with the center server to display the biodata stored in the database.

In the network system, the patient terminal may include a measurement interface connected with at least one sensor for measuring the biodata, a biodata storing section for storing the biodata measured by the sensor and received through the measurement interface, a communicating section for transmitting the biodata stored in the biodata storing section and receiving the patient terminal data from the center server at the time of installation in the patient's home, and an instrument data storing section for storing identification number of each sensor to discriminate the sensor instruments from each other. This allows the patient terminal data to be downloaded from the center server by the patient terminal at the time of its installation, hence eliminating the loading of the patient terminal data to the patient terminal prior to the installation.

In the network system, the patient terminal may performs a procedure of connecting to the center server over the communication network at the time of installation, a procedure of receiving over the communication network, from the center server, patient terminal data which includes the name of the patient corresponding to the identification number of the patient terminal, the identification code corresponding to the patient name, the biodata assigned to the patient, the instrument of the sensor corresponding to the biodata, and the data for controlling the sensor, and a procedure of storing the patient terminal data.

In the network system, the patient terminal may include a measurement interface connected with at least one sensor for measuring the biodata, a biodata storing section for storing the biodata measured by the sensor and received throgh the measurement interface, a communicating section for transmitting the biodata stored in the biodata storing section, an instrument data storing section for storing the identification number of each sensor to discriminate the sensor instruments from each other, and a recording medium interface for receiving the biodata from a detachable recording medium at the time of installation in the patient's home.

In the network system, the patient terminal may perform a procedure of receiving, at the time of installation in the patient's home, from a detachable recording medium the patient terminal data which includes at least one of the name of the patient corresponding to the identification number of the patient terminal, the identification code corresponding to the patient name, the measurement item corresponding to the patient name, the instrument name of the sensor corresponding to the biodata, and the data for controlling the sensor, and a procedure of storing the patient terminal data. This allows the patient terminal data to be received from the recording medium at the time of installation of the patient terminal, hence minimizing the installation period even if the communication speed with the center server is low.

The network system may comprise a doctor terminal for receiving and monitoring a schedule data of the health care action for the patient, a center server for storing the schedule data received from at least the doctor terminal, and a patient terminal for consulting the center server and providing the patient with the schedule data received from the center server. This allows the biodata and schedule data of the patient to be simultaneously monitored or registered by a plurality of members of the medical staff.

In the network system, the patient terminal may have at least a displaying section for displaying the name of the patient, the setting time, and the medical activities in the form of messages and images upon receiving the schedule data or a sound generator for generating a voice sound representing the patient name, the setting time, and the medical activities.

In the network system, the schedule data may include at least one of pairs including a pair of the time and detail of dosage, a pair of the time of visit on the patient and name of a visitor or medical staff, a pair of the time of reservation and detail of the medical treatment at the medical facility, and a pair of the time and detail of measurement of the biodata.

In the network system, the center server may include a homepage data generator for receiving the schedule data from the doctor terminal and converting the schedule data into an HTML or XML format data, and a WEB server for storing the converted data. The patient terminal may have a browser function for communicating with the center server and receiving and displaying the schedule data of the HTML or XML format. This allows the schedule data to be monitored by any personal computer or Internet browser TV which has a browser function.

In the network system, the center server may have a mail transmitting section for storing the schedule data received from at least the doctor terminal and dispatching as an electronic mail the medical activities to be done by the patient at the timing determined by the schedule data. The patient terminal may have a receiver section for receiving the electronic mail from the center server and a displaying section for displaying details of the electronic mail. This allows the schedule data for medical treatment or care to be received by not only the dedicated instrument but also any other instrument such as a personal computer or portable telephone.

In the network system, the patient terminal may have a response entering section for entering activity result indicative whether or not the medical activities of the patient are done according to the schedule data. The center server may have a database for receiving and storing the result of the medical activities from the patient terminal. The doctor terminal may have a section for communicating with the center server to receive the result of the medical activities from the database and displaying the result. This allows the result of medical activities of the patient to be easily monitored by the doctor terminal.

In the network system, the response entering section in the patient terminal may be implemented in an HTML or XML format over the browser. The center server may have a WEB server for communicating with the browser in the patient terminal to receive the result of the medical activities, and a database for storing the result of the medical activities received from the WEB server.

In the network system, the patient terminal may have a mail transmitting section for converting the result of the medical activities into data in a text form and transmitting the text data as an electronic mail. The center server may have an electronic mail receiving section for receiving the electronic mail from the patient terminal, an analyzing section for extracting a text data from the electronic mail to check the result of the medical activities, and a database for storing the result of the medical activities. This allows the result of medical activities of the patient to be monitored by not only the dedicated instrument but also any instrument such as a personal computer or a mobile telephone.

In the network system, the patient terminal may be a mobile telephone or a pager which can transmit and receive the electronic mails. This allows the biodata or schedule data which is different between the patients to be monitored by the mobile telephone or pager even if they are shared by a plurality of patients at the single patient terminal.

### Brief Description of Drawings

Fig. 1 is a schematic view of a medical checkup network system showing a first embodiment of the present invention.
Fig. 2 is a block diagram showing a procedure of action at the patient terminal.
Fig. 3 is a view of a menu selection screen of the patient terminal.
Fig. 4 is a view of a body temperature profile screen of the patient terminal.
Fig. 5 is a block diagram showing a procedure of action at the doctor terminal.
Fig. 6 is a view of a patient selection and menu selection screen of the doctor terminal.
Fig. 7 is a view of a medical advice entry screen of the doctor terminal.
Fig. 8 is a view of an alert setting screen of the doctor terminal.
Fig. 9 is a view of a sensor signal reception sensitivity setting screen of the doctor terminal.
Fig. 10 is a diagram of a procedure of action at the administrator terminal.
Fig. 11 is a -block diagram of the patient terminal.
Fig. 12 is a view of a patient data setting screen of the administrator terminal.
Fig. 13 is a view of a user selection screen of the patient terminal.
Fig. 14 is a view of a health sensor selection screen of the patient terminal.
Fig. 15 is a schematic diagram of a medical checkup network system according to a second embodiment of the present invention.
Fig. 16 is a schematic diagram of a medical checkup network system according to a third embodiment of the present invention.

### Best Mode for Carrying Out the Invention

Preferred embodiments of a medical checkup network system according to the present invention will be described referring to the accompanying drawings.

### First Embodiment.

### <1.1 Configuration of the System>

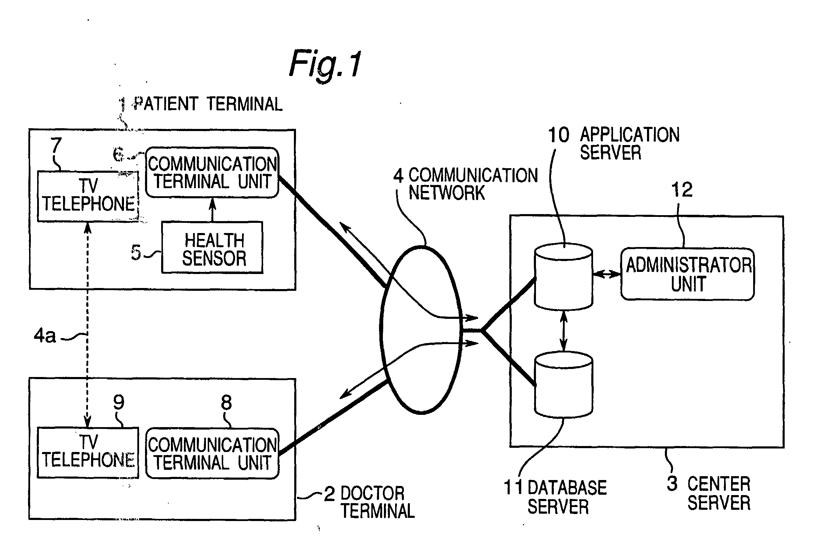

Fig. 1 is a schematic view of the medical checkup network system showing one embodiment of the present invention.

As shown in Fig. 1, the medical checkup network system comprises a patient terminal 1, a doctor terminal 2, and a center server 3, those connected with each other over a communication network 4.

The patient terminal 1 includes a health sensor 5 such as a blood pressure meter or a body thermometer and a communication terminal unit 6 having a browser function. The biodata of a patient measured with the health sensor 5 allows the health of the patient to be checked, and is transferred from the communication terminal unit 6 via the communication network 4 to the center server 3 to store the biodata.

The doctor terminal 2 includes a communication terminal unit 8 having a browser function, which displays the biodata which is measured by the patient terminal 1 and stored in the center server 3 to contribute to a diagnosis. When doctors or medical staff enter advice data of the health care for a patient into the doctor terminal 2 in a form of text or statement, the contents of the data is then transferred and stored to the center server 3. When the medical staff entry and set the schedule data for each patient through the doctor terminal 2, the schedule data of each patient is transmitted and stored to the center server 3. The schedule data is then transferred from the center server 3 to the patient terminal 1. The patient can carry out a series of medical treatments in a schedule managed on time basis according to the schedule data.

A threshold for making a diagnosis of the biodata of each patient can be determined through the doctor terminal. The threshold data is transmitted to the center server 3 to be stored. The threshold or critical level for each patient is determined by the doctor or medical staff through examination so that the patient can properly be classified for emergency treatment.

The patient terminal 1 and the doctor terminal 2 include TV telephones 7 and 9 respectively for permitting a remote diagnosis from real-time video and audio data. When the remote diagnosis is carried out using the TV telephone, the communication network 4 may be replaced by its modification 4a which is different from that of the biodata, depending on transmitting quality.

The center server 3 comprises an application server 10, a database server 11, and an administrator unit 12 for managing these two servers 10 and 11.

The application server 10 stores software for enabling the contents or functions of the patient terminal 1, the doctor terminal 2, and the administrator unit 12.

The database server 11 can store several kinds of data including the biodata received from the patient terminal 1, the advice data and schedule data for health care received from the doctor terminal 2. The database server 11 can also store patient data, patient terminal data, medical staff data, and doctor terminal data received from the administrator unit 12. As the administrator unit can perceive the setting and the operation states of all of the patient terminals and the doctor terminals, the effective management by the center server can be achieved.

Each of the patient data and the doctor data contains access data such as patient ID, patient password, medical staff ID, and medical staff password, thus inhibiting any personnel other than the patients and the medical staff from accessing the biodata of the patient. More specifically, data exchange between the patient terminal 1 and the doctor terminal 2 can be carried out through the center server 3 except during execution of the TV telephone function. This allows the center server 3 to control the data exchange between the patient terminal 1 and the doctor terminal 2 from a single source, hence providing a security controlling means of a certain level. Also, known security technologies may be used, such as encryption of the data to be released over the communication network 4, and provision of a firewall at the entrance of the center server 3. For implementing a higher level of security, the biodata and other confidential data stored in the center server are limited to be capable of being accessed through the patient terminal or the doctor terminal, or by only the patient and the medical staff with their terminals registered, which are registered in advance at the center server 3.

The application server 10 stores content software including patient terminal-use contents, medical tutorial homepages, doctor terminal-use contents, and control terminal-use contents. The content software comprises text files written in the HTML or XML format, video data files including pictures or photos of a JPEG compressed form, or WAV files including digital data of audio signals. The content software can be downloaded by transmitting an URL address signal of the HTTP protocol from the terminal unit.

The patient terminal-use contents may include text files written in the HTML or XML format, JEPG files of pictures and photos. The patient terminal-use contents further may include control programs for processing graphic display of the biodata, interface data with the health sensor 5, JPEG files of images taken by a video camera, WAV files of the biodata changing on time-basis transferred from a stethoscope or an electrocardiograph, execution of controlling the TV telephone, or the like. Also, the patient terminal-use contents may include terminal control information for implementing the function of the patient terminal 1. The terminal control information may include a patient terminal control data such as a sensitivity setting data for the health sensor, received from the doctor terminal 2 and stored in the center server 3, and a communication address data. The communication address data includes a network address data of telephone numbers and IP addresses received from the administrator unit 12 for connection with the Internet, and the telephone number of the doctor terminal 2 which is a destination of communication via TV telephone.

This allows the patient terminal of each patient to be remotely controlled by the administrator unit, hence enabling the monistical management and maintenance of the medical checkup network system at higher efficiency. Also, the text file includes medical support information such as the advice data and schedule data for health care received from the doctor terminal 2.

The application server 10 may include a software for accessing the database server 11, a graphic software for displaying a graphic profile of the biodata on the patient terminal 1 or the doctor terminal 2, an alert determination software, and a patient data transfer software. Those softwares are written in a specific language such as Java Servelet. The alert determination software is designed for detecting that the biodata of a patient received from the patient terminal 1 exceeds an alert setting level determined by the doctor terminal 2, and notifying the doctor terminal 2 of the fact. The patient data transfer software is designed for storing the advice data and schedule data for health care received from the doctor terminal 2 and the control data for the patient terminal 1 such as the sensitivity data of input signals from the health sensor 5, and downloading those data into the patient terminal 1 when the patient terminal 1 turns on.

The database server 11 is designed for storing a variety of medical data received from the patient terminal 1 and the doctor terminal 2, and permitting only specific terminals registered in advance to access the data. The database server 11 may include contents software such as a registered user data, a software version management data, the patient terminal data, biodata files, and medical support data files.

The registered user data includes data relating to registered patients, the doctors, nurses, and relevant medical staff, the clinics and hospitals, and the system supervisors to be registered.

The software version management data may include data relating to a software version and data indicative of reception (or installation) of software for each patient terminal.

The patient terminal management data may include data relating to a patient terminal system, data relating to a registered measuring sensor, registered user data for each patient terminal, and measurement item data for each patient.

The biodata file may include the biodata of each patient measured by the health sensor 5, the biodata added with date/time data for each item.

The medical support data file may include the advice data and schedule data for health care of each patient received from the doctor terminal and added with date/time data.

### <1.2 Operation of the System>

### (Operation at the Patient Terminal)

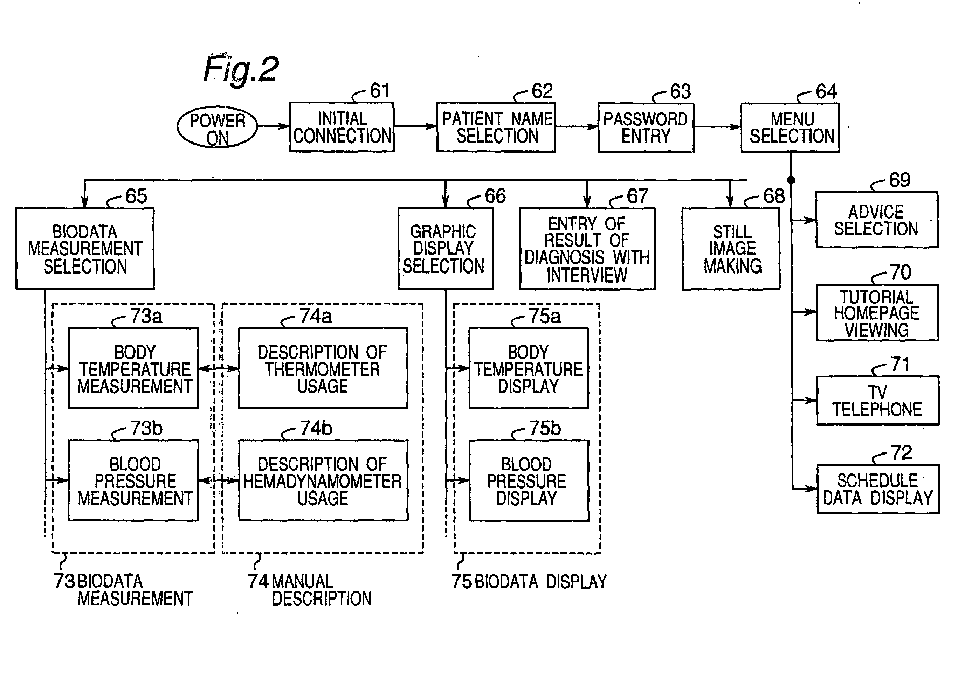

Fig. 2 is a diagram showing a procedure of action at the patient terminal 1.

When the patient terminal 1 is energized, the communication terminal unit 6 communicates with the center server 3 over the communication network 4 for enabling an on-line operation to execute an initial connection process 61. More particularly, the initial connection process 61 includes updating the contents software on the patient terminal 1, checking reception of the medical support data, checking new reception of advice data from the medical staff, and processing un-transferred data including biodata measured by the patient terminal. This allows the patient to update the functions of the patient terminal before use of the patient terminal.

The updating of the software includes, connecting to the center server 3, comparing the version data of the software stored in the patient terminal 1 with a latest version stored in the database server 11, and then if they are different, downloading the latest version to be replaced. The software installed in the patient terminal 1 is optimized for each patient and is distinguished for each patient. That is, the latest version means the newest version for a patient. As the contents such as the functions of the patient terminal 1 can be modified by the center server 3, the overall operation of the terminal can be improved in the efficiency. The patient terminal 1 has a function of comparing the software in use with a latest version stored in the center server 3, and updating the version of the used software with the latest version if both versions are different.

The checking new reception of the advice data from the medical staff is carried out using a flag provided in the advice file received from the medical staff registered at the database server 11 of the center server 3 in the following manner.

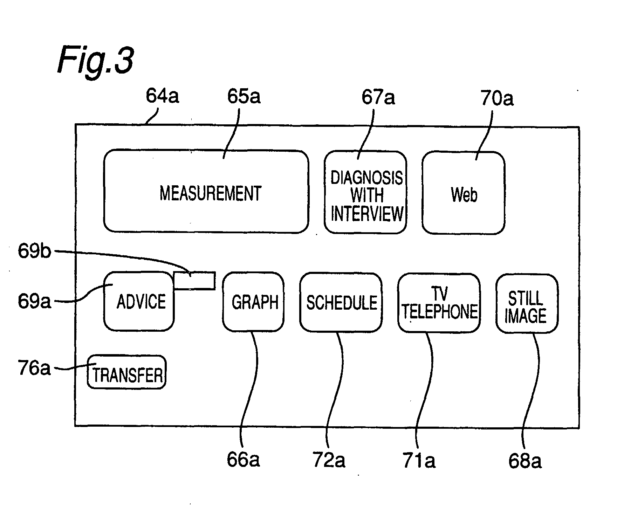

In the checking new reception, the patient terminal 1 resets the flag when the advice data is received from the medical staff, and then turns on a reception marking 69b displayed on a menu selection GUI screen 64a shown in Fig. 3 for indicating to the patient that the advice data is new received. The patient can know easily arrival of the advice data sent from medical staff such as the doctor. It means that the patient can get the advice data at an appropriate timing, thus improving the effect of the medical treatment.

The processing un-transferred data is explained below. In common, after the entry of the biodata is completed, such as measurements from the health sensor 5, response to a diagnosis question, and still images taken with a video camera, a transfer button 76a displayed on the menu selection GUI screen 64a shown in Fig. 3 is pressed automatically enabling the connection with the network, and then the biodata is transferred to the center server 3. The processing un-transferred data is executed for transmitting the data which has not been transferred yet at the next energization of the terminal, when the operation of pressing the button has not been executed. This allows the biodata, the result of a diagnosis with an interview, and other patient data to be bundled and transmitted at once to the center server 3, hence improving the efficiency of the use of the communication network.

After the initial connection process 61 is completed, the terminal is automatically disconnected to be an off-line state and the procedure goes to a patient name selection process 62. As the on-line operation and the off-line operation are automatically switched from one to the other according to the type of the process, the communication network can be used at higher efficiency without trading off the functions. It is useful in that a telephone line can be shared, since usually one telephone line is provided with one house.

The patient name displayed in the patient name selection process 62 is registered through the center server 3 in advance, and can thus be displayed as well as number of people specifically at each patient terminal. The patient terminal data stored in the center server 3 may include not only an official name of each patient but also unofficial proper name such as a nickname. The patient name data is constantly updated by the software updating process in the initial connection process 61.

When the patient name to use is determined in the patient name selection process 62, the procedure goes to a password entry process 63. The password is entered using the numeral keys 0 to 9 of a GUI touch keyboard. The maximum number of figures of the password is within 10 and is usable arbitrarily. The number of figures may also used for identification data.

The entered password is then compared with the password registered for each patient. If the passwords are matched, the procedure goes to the following process. The password data of each patient, as same as the patient name data, is systematically updated by the software updating process in the initial connection process 61.

The registration of the password data may be carried out at a password registration process separately provided.

As the entered password is correct, the procedure goes to a menu selection process 64. Fig. 3 shows one embodiment of a GUI operation screen 64a, where touch keys (65a, 66a, 67a, 68a, 69a, 70a, 71a, and 72a) for selection of eight different functions are disposed together with the GUI button images.

Using these touch keys(65a, 66a, 67a, 68a, 69a, 70a, 71a, and 72a), one of the following processes is selected and activated: selection of biodata to be measured 65; selection of graphic display 66; entry of result of diagnosis with an interview 67; capturing still image 68; advice selection 69, tutorial homepage viewing 70; TV telephone 71; and schedule display 72.

When the biodata measurement selection process 65 is selected, biodata desired to be measured can be selected, using the touch keys of the GUI screen to select biodata measurement items (biodata to be measured). The biodata measurement items may include a body temperature, a blood pressure, a blood sugar, a weight, a stethoscope checkup, and an electrocardiogram, each corresponding to a respective health sensor.

The biodata measurement items are determined by the administrator unit 12 of the center server 3 depending on the state of the patient for each patient. The GUI screen for the biodata measurement selection process is individually attributed to each patient.

After the biodata measurement items are determined, the procedure goes to a biodata measurement process 73 for performing measurement with the health sensor 5 and entry of the measurements.

When the graphic display selection process 66 is selected, biodata display items (biodata to be displayed) are determined by operating the GUI screen to select the biodata display items. The biodata display items relates to the biodata measurement items.

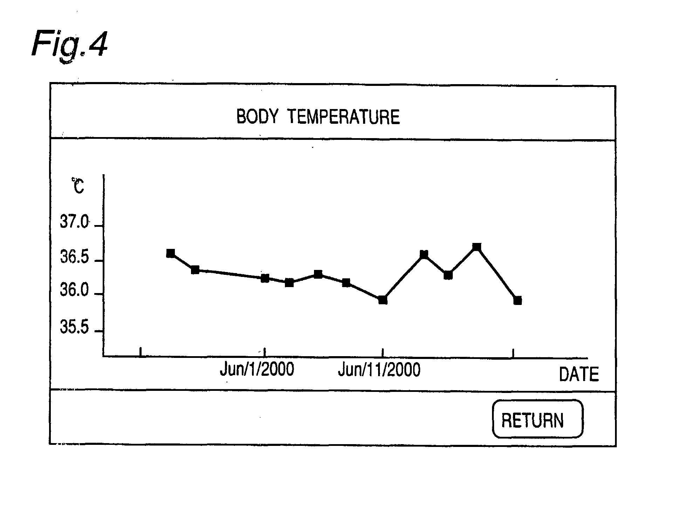

As the biodata display items are determined, the procedure goes to a biodata display process 75 where a graphic profile on time basis of the biodata is displayed. Fig. 4 illustrates an example where the biodata is a body temperature.

When the advice display process 69 is selected, a list of titles of advice mails is shown in the GUI screen. When one title in the list is selected by touching, the advice mail corresponding to the selected title is open to be readable.

When the tutorial homepage viewing process 70 is selected using one touch key, the terminal is automatically connected to the URL address of a homepage provided by the predetermined medical sector for displaying contents relating to health teaching.

When the schedule display process 72 is selected, a schedule table for the medical treatments on the current day is displayed. The schedule data is entered by the doctor or medical staff and transmitted from the doctor terminal 2 to the database server 11 in the center server 3. The schedule data is also transferred to the patient terminal 1 when the initial connection process 61 performs the software updating action.

### (Operation at the Doctor Terminal)

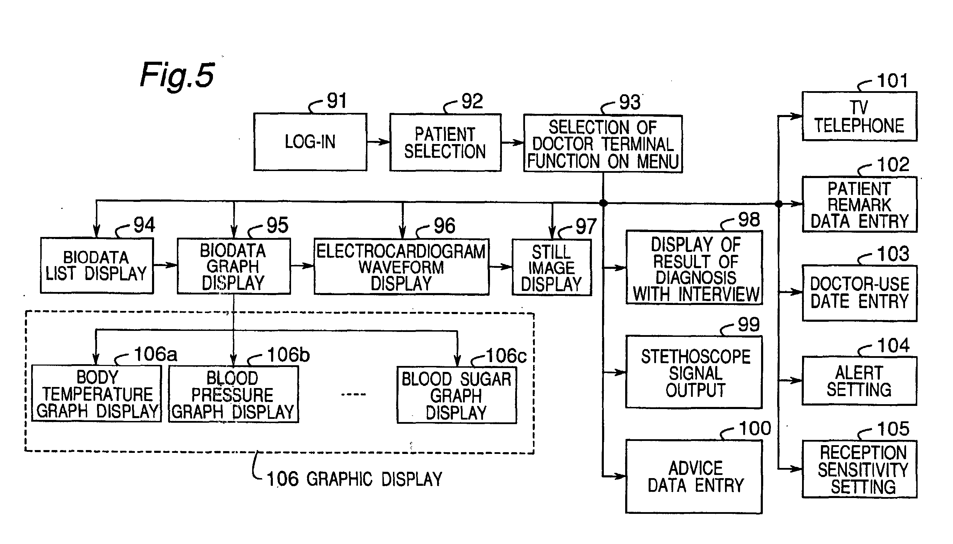

Fig. 5 shows a procedure of action at the doctor terminal 2.

When the doctor terminal 2 is energized, a log-in process 91 has first to be carried out. When the ID code and the password registered in the center server 3 are entered, the terminal is ready for the operation.

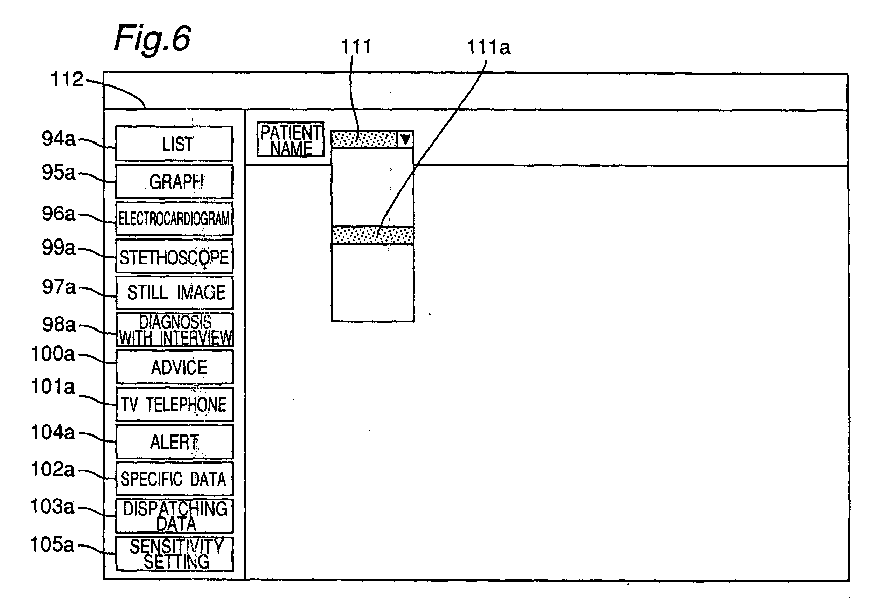

After passing the log-in process 91, a patient selection process 92 is executed, where the name of a patient 111a to be examined is selected by a method of pull-down menu such as a window 111 to entry a patient name shown in Fig. 6.

Then, a menu selection process 93 of functions of the doctor terminal is carried out by clicking a function button (94a to 105a) provided in a menu selection area 112 of Fig. 6.

The functions of the doctor terminal may include biodata list display 94, biodata graphic display selection 95, an electrocardiogram waveform display 96, still image display 97, display of result of a diagnosis with an interview 98, a stethoscopic sound output 99, advice entry 100, TV telephone 101, special patient data entry 102, doctor data entry 103, alert setting 104, and reception sensitivity setting 105 for health sensor signal.

In the biodata graphic display selection process 95, desired biodata are selected, and then the graphic display process 106 corresponding to the selected biodata is carried out for providing a graphic representation of the biodata.

The patient selection process 92 can be executed any time on the function screen of Fig. 6.

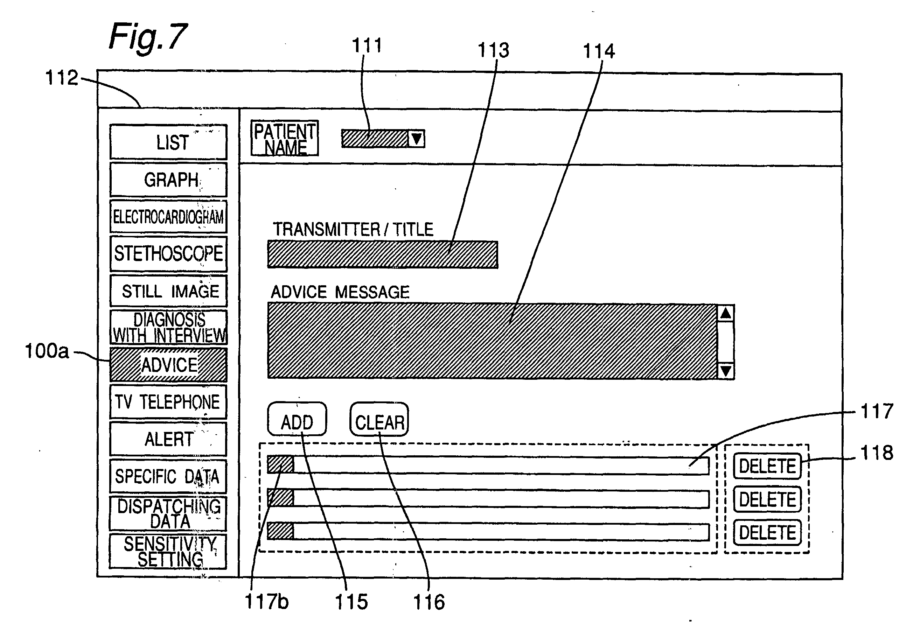

Fig. 7 illustrates a display screen for carrying out the advice entry process 100. A color of a selected button 100a for selecting the advice is turned to be of a color different from other color of buttons which are not selected so as to easily recognize that the button is selected. The patient name selection window 111 displays a destination name of the patient to which the medical advice is transmitted.

Using a keyboard of the doctor terminal 2, the name of a sender and the tile of advice can be entered through a window 113 for entry of sender name, and a text of the advice can be entered through a window 114 for entry of the advice. Also, the advice text entered through the advice text entry window 114 can be transferred to the center server 3 when an extra button 115 is pressed. The advice text being created can be deleted by pressing a clear button 116. The transferred advice text is stored to the database server 11 of the center server 3, and transferred to the patient terminal 1 as required.

The title display window 117 displays the titles of the advice texts in order of recency of the advice. Each advice text displayed in the screen is accompanied with a delete button 118, and can thus be deleted from the center server 3 by pressing the delete button 118.

Also, a title display box 117 may include a confirmation mark 117b at the front end. The mark 119 indicates that the advice text has been received by the destination patient terminal. It may be nonsense if the patient receives an advice from the medical staff but fails to read it. However, the medical staff can recognize whether or not the advice text is correctly received by the destination patient, and then provide properly subsequent services.

The confirmation mark 117b may simply be implemented by a flag set on the database server 11 upon the advice text being received by the patient terminal 1.

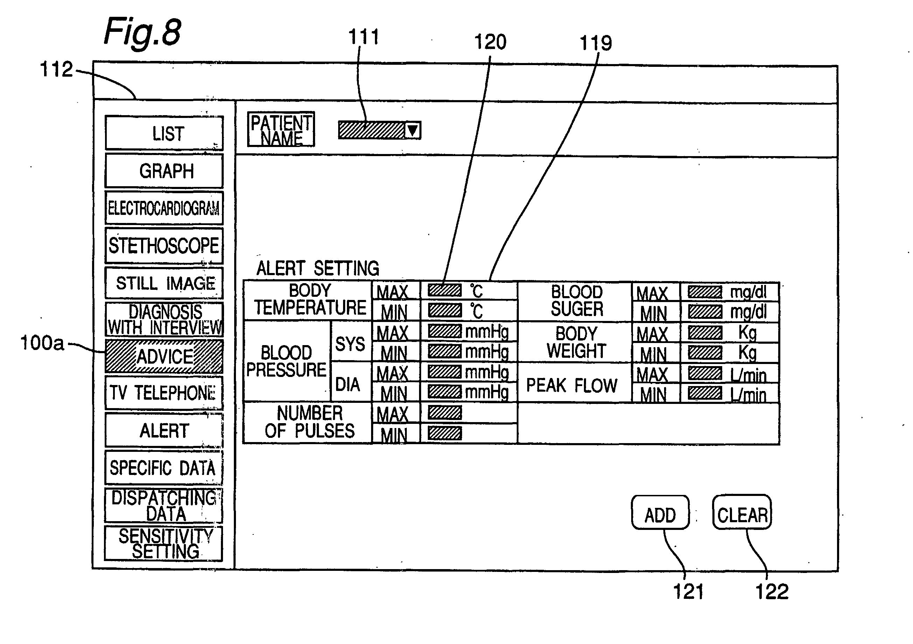

Fig. 8 illustrates a display screen for carrying out the alert setting process 104. When an alert setting button 104a in the menu selection area 112 is turned on, its color is changed for ease of indication of selection.

The patient selection box 111 displays the name of a patient to which the advice is delivered.

In an alert setting table 119, the maximum threshold and the minimum threshold of each biodata can be entered in the setting box 120. When the measurement of the biodata exceeds the setting values displayed in the setting box 120, an alert is released. Those alert setting data can be transmitted to the center server 3 by pressing an addition button 121. When a clear button 122 is pressed, the alert setting data can be deleted. Since the alert level is determined by the family doctor or medical staff according to a result from diagnosis of conditions of the patient, appropriate critical level of the patient can separately be notified.

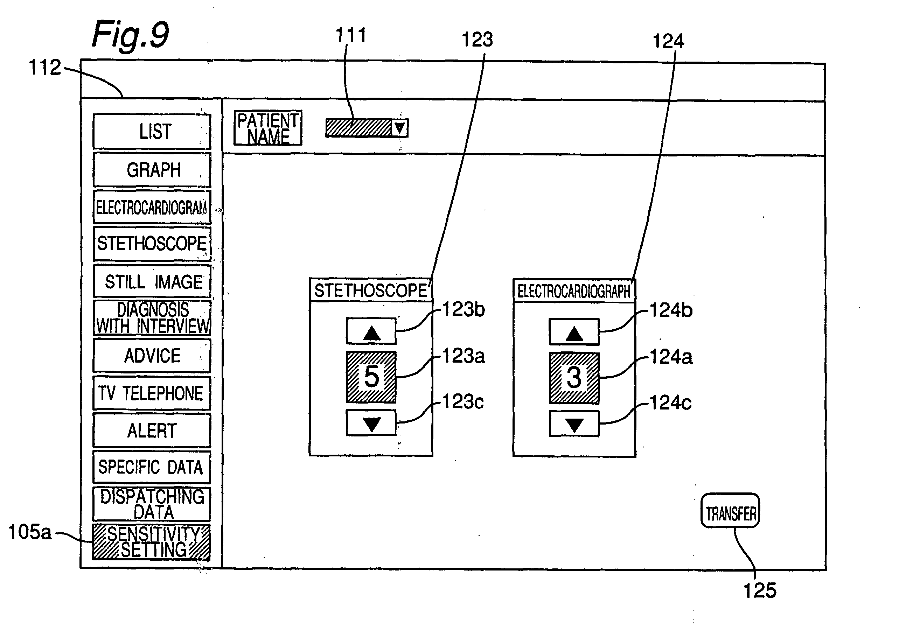

Fig. 9 illustrates a display screen for carrying out reception sensitivity setting 105 for health sensor signal. When a button 105a for setting health sensor signal reception sensitivity in the menu selection area 112 is switched on, its color is changed for ease of recognition.

The patient name selection box 111 displays the name of a patient using the patient terminal 1. A stethoscope setting box 123 includes an area 123a for displaying a setting level, an upward key 123b, and a downward key 123c. An electrocardiograph setting box 124 includes an area 124a for displaying a setting level, an upward key 124b, and a downward key 124c for entering desired settings of the sensitivity. A transfer button 125 is provided for transferring the setting data to the center server 3. The sensitivity setting data received by the center server 3 is stored in the database server 11 and, if desired, delivered to the patient terminal 1.

In the patient terminal 1, the sensitivity setting data is fed to an amplifier (not shown) receiving a health sensor signal for adjusting the level of health sensor signal.

### (Operation of Control Terminal Unit)

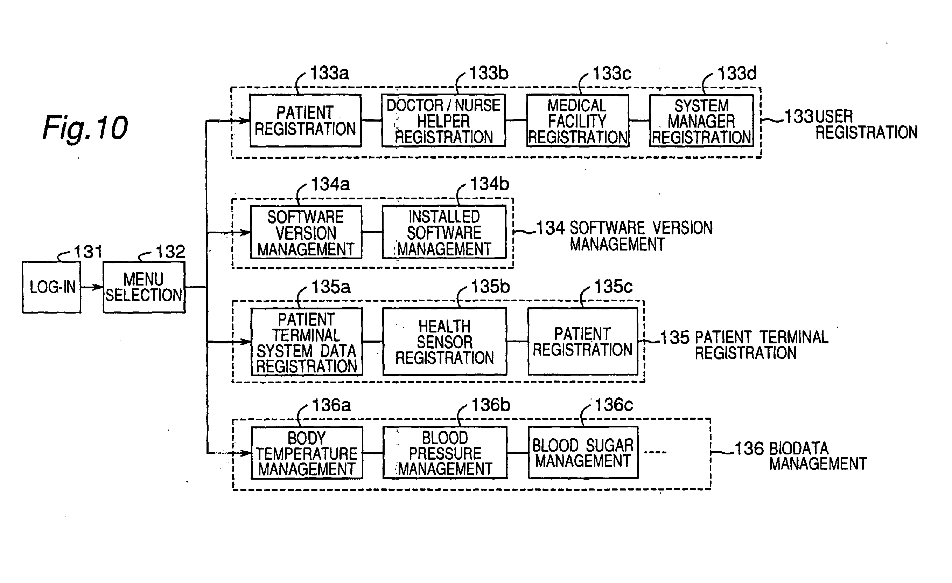

Fig. 10 is a diagram showing a procedure of action at the administrator unit 12.

When the administrator unit 12 is energized, a log-in process 131 has first to be carried out. As the ID code and the password registered in the center server 3 are entered, the terminal unit is ready for the operation. Since the registration on the center server 3 is carried out through the administrator unit 12, an initial operation of the log-in process can be conducted using a provisional ID code and a provisional password only at the fist time. The log-in process 131 is followed by a menu selection process 132 for selecting the function of the administrator unit 12. Four different functions are selected, including user registration 133, software version management 134, patient terminal registration 135, and biodata management 136. Those functions are to manage the contents software stored in the database server 11.

The user registration 133 comprises a patient registration process 133a, a doctor/nurse/care person registration process 133b, a medical facility registration process 133c, and a system manager registration process 133d. Each process registers access right of the user, the medical staff, or the medical facility. Each of these registered data is efficiently related and stored to the database server 11 which is a relational database, and thus can be used efficiently in the patient terminal and the doctor terminal. Only the user, medical staff, and medical facility registered by this registration processs can be allowed to use the medical checkup network system. Since the particular system manager who is allowed in advance registers or cancels properly in a strictly controlled predetermined manner, it becomes possible to provide the monistical security management at a higher level.

The software version management 134 comprises a software version data management process 134a and a software reception data management process 134b at the patient terminal of each patient. In the software version data management process 134a, version data of the contents softwares supplied from the application server 10 for each terminal is managed, and thus it is possible to know the latest state of the version. In the software reception data management process 134b, versions of the software in service at the terminal can be recognized.

The patient terminal registration process 135 comprises a patient terminal system data registration process 135a, a health sensor registration process 135b, a patient registration process 135c for each patient, and a measurement item registration process 135c for each patient.

The biodata management process 136 is designed for carrying out maintenance of the biodata measured by the health sensor 5 and stored. For example, the process deletes biodata received falsely from the patient terminal 1.

According to the foregoing arrangement of the present invention, the biodata of each patient can readily be accessed by the medical staff thus permitting the medical treatment to be conducted quickly and efficiently. According to the arrangement, the patient can daily check his biodata, have the biodata monitored daily by its doctor or nurse, and thus get a high satisfaction. The biodata of each patient can be viewed and checked by a plurality of specialists each having respective medical field, and hence the diagnosis becomes more accurate. Since medical support data including the advice data and the schedule data from the medical staff can be transferred for each patient, it is possible to deal with the patient fine. Also, as the sensitivity of health sensor signal reception at the patient terminal is controlled from remote locations, the measurement of biodata can be increased in the accuracy. This function is particularly desirable because the stethoscope signal and the electrocardiograph signal are different in the magnitude between individual patients. The brightness or focusing of a video camera, the sensitivity of a microphone, and the output of a loudspeaker may also be controlled by the same manner.

### (Configuration of the Patient Terminal)

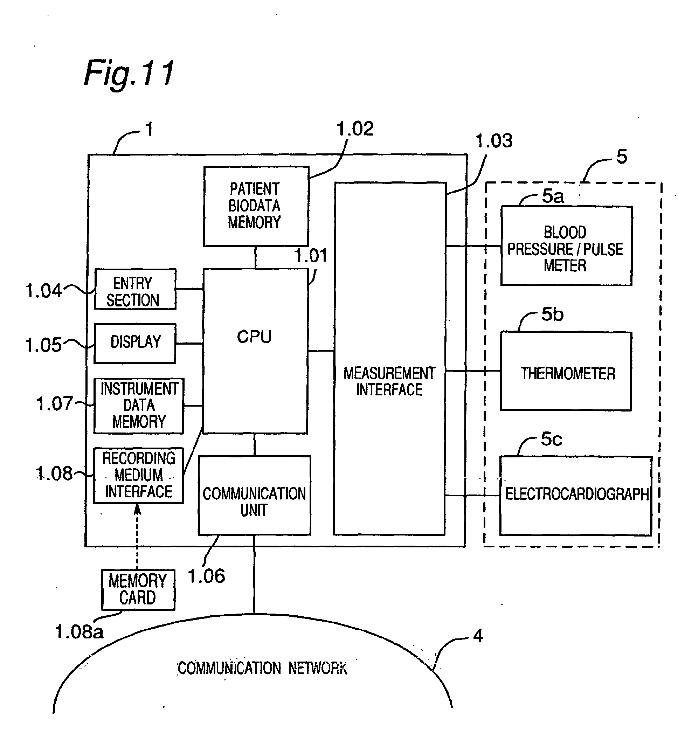

Fig. 11 is a block diagram of the patient terminal 1.

The patient terminal 1 comprises a central processing unit 1.01 (referred to as a "CPU" hereinafter) for controlling the operation of each component and the exchange of data, a health sensor 5, a patient biodata memory 1.02 for storing the measured biodata, a measurement interface 1.03 for communication with the health sensor 5, an entry unit 1.04 comprising a touch panel or a keypad for selecting on the menu and entering data, a display 1.05 comprising a liquid crystal display for displaying the entered data and the measured biodata, and a communication section 1.06 for communicating with the center server 3 over a communication network 4 such as a public circuit.

The health sensor 5 incorporates a blood pressure/pulse meter 5a, a body thermometer 5b, and an electrocardiograph 5c. The patient terminal 1 also includes an instrument data memory 1.07 for storing a serial number of the terminal 1 as an identification number. Since the serial number stored in the instrument data memory 1.07 is usually assigned to each patient terminal and stored in a nonvolatile memory at manufacturing process, the serial number can identify surely the patient terminal.

### (Method of Setting Patient Terminal Data)

A method of setting the patient terminal data in the medical checkup network system of the present invention will be described in more detail. The description is made to, as an example, a case that three members in Yamada's family including "Taro Yamada", "Hanako Yamada", and "Ichro Yamada" are registered to one patient terminal 1.

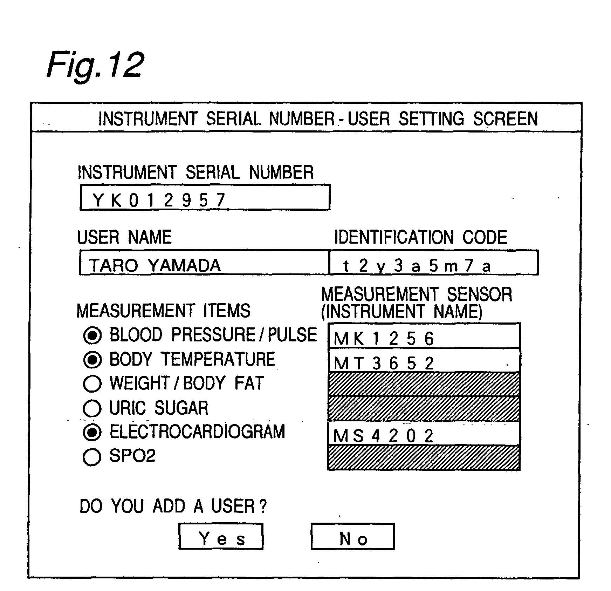

Fig. 12 illustrates a machine serial number/user setting screen of the control terminal 12.

First, the data about each patient is entered in relation to the serial number of the patient terminal 1. For example, for Mr. Taro Yamada as shown in Fig. 12, on the setting screen with the serial number "YK012957", "Taro Yamada" and "t2y3a5m7a" are entered as a patient name and an identification code, respectively, and then items to be measured are selected from the measurement items. In Fig. 12, three conditions to be measured are selected, including "blood pressure/pulse", "body temperature", and "electrocardiograph record". Also, entering the name of each instrument of the health sensor (measuring sensor) allows the respective measurement sensor setting program to be systematically specified.

The measurement sensor setting program is designed for providing a screen for selecting and setting the health sensor 5 and retrieving and displaying the measurement data. The programs are stored in the center server 3 for each name of the instrument of the health sensor. The program to be required is downloaded from the center server 3 to the patient terminal 1 when the patient terminal 1 is implemented or when the health sensor is added or changed.

Afterward, to add the other patients "Hanako Yamada" and "Ichoro Yamada" to the patient terminal 1, a "yes" button next to a message "Want to register another user?" is pressed and the same steps as those described above is taken, thereby the patient terminal data being set.

As described above, the patient terminal data can be registered into the center server 3 by entering the patient name, the identification code, the measurement items, and the instrument name of the health sensor, which correspond to the instrument serial number.

Next description is made to an operation in which after the registration, the patient terminal 1 identified by the serial number "YK012957" which is registered in the instrument data memory 1.07 is installed in the Yamada family.

When the patient terminal 1 is connected to the communication network 4 such as the public circuit to be activated, the CPU 1.01 is connected to the center server 3 via the communication section 1.06 and the communication network 4. The CPU 1.01 reads out the serial number "YK012957" from the instrument data memory 1.07 and transfers it to the center server 3 over the communication network 4. In response, the center server 3 dispatches the patient terminal data specified by the serial number to the patient terminal 1.

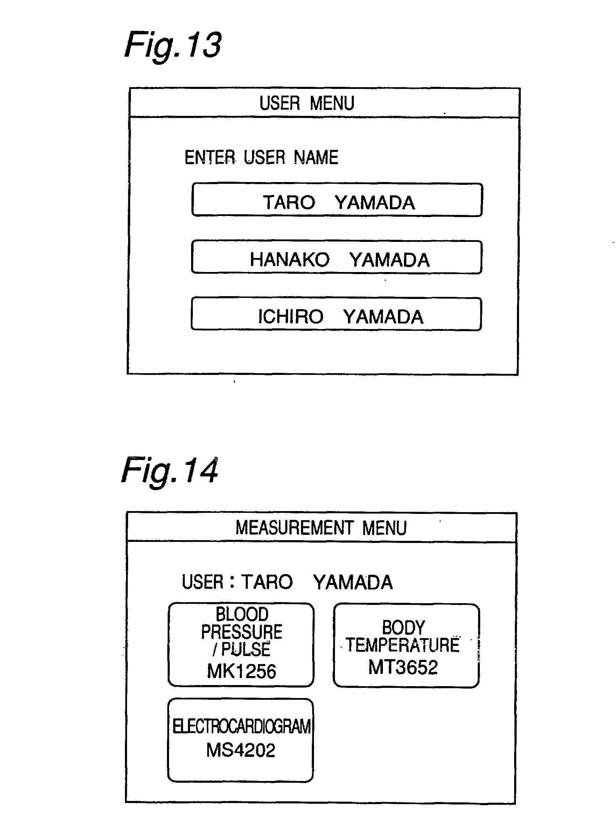

The patient terminal 1 upon receiving the patient terminal data from the center server 3 displays the menu screen, as shown in Fig. 13, on the display 1.05 for selection of the patient. The name of the patient to be measured is selected by operating the touch panel of the entry unit 1.04, and then the identification code is entered from an identification code entry screen (not shown) which is displayed after the selection of the patient. Thus, the patient is identified and the menu selection screen shown in Fig. 3 appears.

When the measurement key 65a in the menu selection screen of Fig. 3 is pressed, the health sensor selection screen is displayed as shown in Fig. 14. As apparent, the setting indicates that for the member "Taro Yamada", " blood pressure/pulse", "body temperature", and "electrocardiogram " are set to be items to be measured. The instrument names in the health sensor 5 corresponding to the items to be measured are also displayed, which indicates that the corresponding measurement instrument control programs are received. As described above, entry of the name and the identification code of each patient allows the patient terminal 1 to be shared by a plurality of members without confusing the data. Also, the biodata of each member is protected from any other members and will be kept with high secrecy. The patient terminal according to the present invention can receive the programs required for the health sensor used by the pertinent, by specifying the patient for each patient terminal at installation of the patient terminal, and thus can provide the patient terminal which has a best setting the patient.

In the above description, the measurement instrument setting data in the patient terminal data according to the present invention is a program for controlling the operation of the measuring instrument. The setting data however may be an program ID for specifying and authorizing the program for controlling the operation of the measuring instrument. This allows only the program which is required by the patient out of programs stored in the patient terminal in advance to be authorized to be used or to be given with access right. As a result, the patient terminal needs not to download a large volume of the programs from the center server 3 at the time of installation, and the installation will be completed shortly.

### (Setting with Memory Card)

Another method of setting the patient terminal data may be provided using a memory card 1.08a which is a type of recording medium arranged detachable for storing the patient terminal data. The memory card 1.08a is loaded to a memory interface 1.08 for connection with the network to receive the patient terminal data. System including the memory card 1.08a is designed so that the data stored in the card can be read from the card 1.08a only when the instrument serial number stored in the card 1.08a is identical to that of the patient terminal 1 which requests the data. This inhibits the patient terminal data from being assigned to an unauthorized patient terminal. The patient terminal data is stored in the center server 3, and is generated by a memory card writer connected to the center server 3.

When the patient terminal 1 loaded with the memory card 1.08a is energized, the CPU 1.01 examines through the memory interface 1.08 whether or not the patient terminal data corresponding to the instrument serial number received from the instrument data memory 1.07 is stored in the memory card 1.08a. When the patient terminal data is stored in the memory card 1.08a, the patient terminal 1 reads out the patient terminal data from the memory card 1.08a. The procedure after the reading out of the patient terminal data is identical to that for receiving the patient terminal data over the communication network 4.

Thus, the patient terminal can use the memory card prepared at the installation in advance to identify the patient and the requirements for operation. Accordingly, the patient and the requirements for operation can easily be set at the installation.

### Second Embodiment.

### <2.1 Configuration of the System>

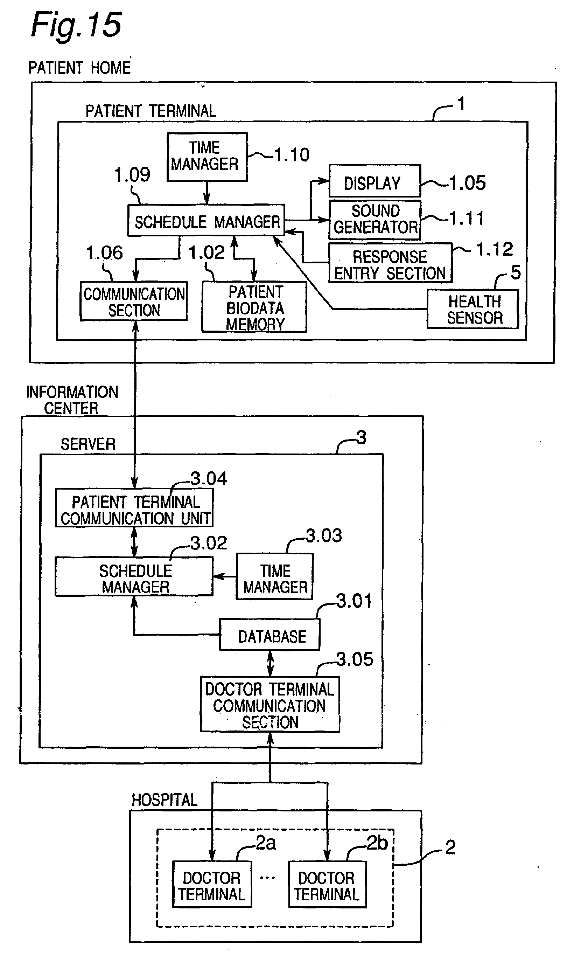

Fig. 15 is a schematic diagram of a medical checkup network system according to the second embodiment of the present invention. In the network system of this embodiment, the patient terminal 1 has a function for proposing to the patient the schedule data received from the center server 3, and prompting the patient to enter the result of medical activities executed based on the schedule data.

The schedule data in this embodiment means a set of medical activities executed at predetermined time and for a predetermined interval. The medical activities are:
1) instruction for dosage to the patient (dosage amount, time, types of drags),
2) time and task of a visit of the doctor, nurse, or medical staff on the patient (visiting time and visitor's name, task),
3) reservation time for attending the clinic or hospital, or reservation time for diagnosis,
4) duration of measuring the biodata including blood pressure, body temperature, and electrocardiograph output (measuring time and measuring items).

As shown in Fig. 15, the patient terminals 1 comprises a communication section 1.06 for communication with the center server 3, a schedule manager 1.09 for managing the schedule data of each patient and instructing to output video data and audio data at a predetermined time, a patient biodata memory 1.02 for storing the schedule data and the biodata for a plurality of patients, a time management unit 1.10 for managing time, a display 1.05 for displaying images and texts determined by the schedule manager 1.09, a sound generator 1.11 for emitting voice, effect, and music sounds determined by the schedule manager 1.09, a response entry section 1.12 for entry of the result of actions determined by the schedule data for the schedule data outputted from a -display 1.05 or a sound generator 1.11, and a health sensor 5. A plurality of patient terminals 1 may be connected to the single center server 3.

The center server 3 comprises a database 3.01 for storing all relevant data of patients including the schedule data, the biodata, and the name and address data, a schedule management unit 3.02 for retrieving the schedule data of each patient from the database 3.01 to transmit the data to the patient terminal 1 and for registering the result of actions based on the schedule received from the patient terminal 1 into the database 3.01, a time management unit 3.03 for monitoring the current time, a patient terminal communication unit 3.04 for communication with the patient terminal 1, and a doctor terminal communication section 3.05 for communication with the doctor terminal 2. The functions of the center server 3 can be implemented by control programs and database softwares stored in the application server 10 and the database server 11 shown in Fig. 1.

The doctor terminal 2 comprises a common computer such as a personal computer or workstation which has a display function with a display, an entry function with a keyboard or a mouse, and a communication function. The doctor terminal 2 communicates with the doctor terminal communication section 3.05 in the center server 3 for receiving the schedule data of each patient and displaying the biodata of the patient and the result of actions executed according to the schedule data.

### <2.2 Operation of the System>

An exemplary action of the medical checkup network system of this embodiment will be descried in a sequence.

### (Step 1):

The medical staff enter the schedule data of each patient at the doctor terminal 2.

The medical staff including doctors, nurses, and care people enter a schedule data as denoted below, from the doctor terminal 2 and register the data into the database 3.01 in the center server over the communication line.

### -- Example of Entry --

- Entry by doctor = patient A : "dosage of three tablets of drag A at 12:00 every day".
- Entry by nurse = patient A : "measurement of body temperature at 7:00 every day".
- Entry by care person = patient A : "visit home (scheduled) at 13:00 on 25th of March".

The above example exhibits the respective schedule data of patient A created by the doctor, the nurse, and the care person, separately. Those entries can be registered from a plurality of doctor terminals 2 (2a, 2b...) in a hospital.

In the prior art, the schedule data of each patient is stored in its related single doctor terminal and can hardly be modified from other doctor terminals. This embodiment allows the schedule data of each patient to be stored together with the biodata in the database 3.01 of the center server 3 and hence enabled to be accessed from the other doctor terminals. Also, an extra schedule data may be made and registered by any of the other doctor terminals.

### (Step 2):

The center server 3 transfers the schedule data from the database 3.01 to the patient terminal 1.

Relationship between name of a patient and a patient terminal 1 operated by the patient is stored in the database 3.01. The schedule management unit 3.02 in the center server 3 transfers the schedule data of each patient to the registered terminal through the patient terminal communication unit 3.04.

The transmission of the schedule data from the center server 3 to the patient terminal 1 may be implemented by one of the following three manners:

### a) Periodic center polling

The schedule management unit 3.02 receives the current time from the time management unit 3.03, and dispatches in a lump the schedule data of the patient at a predetermined time set in advance. By repeating this action, the schedule data can be transmitted periodically (e.g. once a day at 12:00 midnight);

### b) Seriatim center poling

The schedule management unit 3.02 receives the current time from the time management unit 3.03, calls through the patient terminal communication unit 3.04 the patient terminal just on or slightly earlier than the setting time determined by the schedule data, and then dispatches the schedule data of the patient at that time. For example, in case that the schedule data indicates "patient A, dosage of three tablets of drag A at 12:00 every day", the center server 3 daily calls the patient terminal at 12:00 to dispatch a message of "patient A: dose with three tablets of drag A";

### c) Data reception with a call from patient terminal

The schedule manager 1.09 of the patient terminal 1 receives the current time from the time management unit 1.10, and calls the center server 3 through the communication section 1.06 at a predetermined time to connect with the server 3. In response, the schedule management unit 3.02 of the center server 3 confirms the connection with the patient terminal 1 and then transmits the schedule data from the database 3.01 to the patient terminal 1.

Alternatively, the communication section 1.06 may call the center server 3 systematically when the patient terminal 1 is powered on to establish the connection, and schedule data may be received at that time.

Throughout the manners a) to c), the schedule data dispatched from the center server 3 may include not only time and text data but also audio and video data.

The schedule data dispatched to the patient terminal 1 by any one of the above manners is then stored in the patient biodata memory 1.02 by the schedule manager 1.09.

### (Step 3):

The patient terminal 1 notifies the schedule data to the patient in the form of messages, images, and sounds when a setting time is up.

More specifically, the schedule memory 1.09 receives the current time from the time management unit 1.10 and compares the current time with the setting time of the schedule data stored in the patient biodata memory 1.02. When the current time is identical to (or slightly earlier than) the setting time in the schedule data, the schedule manager 1.09 displays the schedule data in the form of messages and images on the display 1.05 and simultaneously drives the sound generator 1.11 to output related sounds and music. The output sound may be generated by a sound synthesizing process in the schedule manager 1.09 or selected from a group of predetermined audio messages.

### (Step 4):

The result of medical activities executed according to the schedule data is entered by the patient.

More particularly, the result of medical activities on the above (Step 3) carried out by the patient according to the timetable provided from the display 1.05 and the sound generator 1.11 is entered from the response entry section 1.12. The response entry section 1.12 comprises mainly a keypad, a keyboard or a pointing device, and can be used for entry of information indicative whether the patient executes the activities according to the schedule. When a result of medical activities which is executed according to the schedule data is entered, the schedule manager 1.09 drives the display 1.05 and the sound generator 1.11 to release the messages, images, sounds, and so on for prompting the patient to enter the result.

The result of medical activities is then stored in the patient biodata memory 1.02 and transferred from the communication section 1.06 to the center server 3. The result may be transmitted at the entrance, or at the next timing of transmission of the scheduled data.

By conducting the above process, the schedule data of each patient generated by the doctor terminal 2 can timely be received and displayed on the patient terminal 1. Also, as information indicative whether medical activities determined by the schedule data is executed or not is entered by the patient, the result can readily be monitored at the doctor terminal 2.

### Third Embodiment.

### <3.1 Configuration of the System>

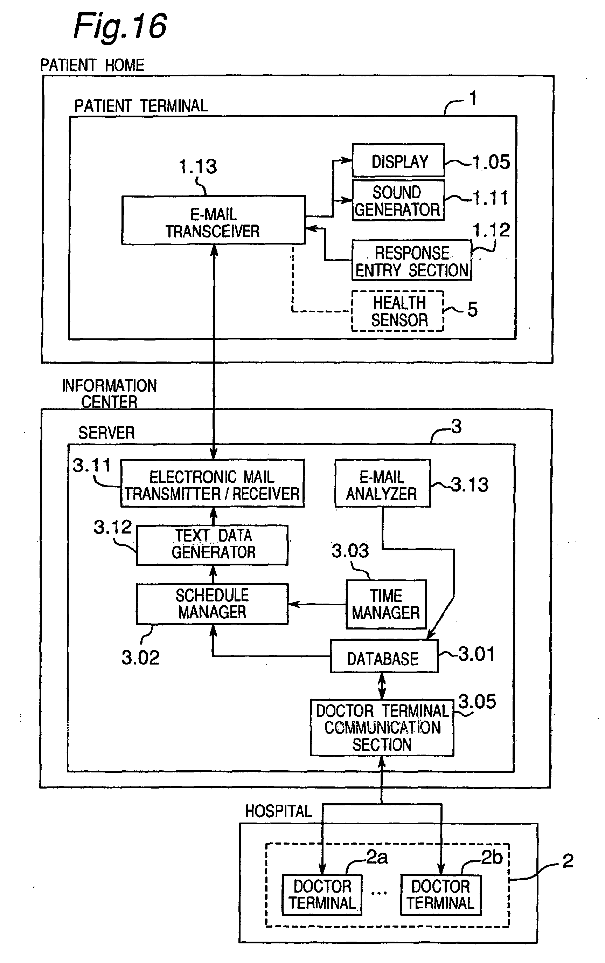

Fig. 16 is a schematic diagram of a medical checkup network system according to the third embodiment of the present invention.

In this embodiment, the patient terminal 1 includes an E-mail transceiver 1.13 for exchanging electronic mails (E-mails) with the center server 3.

The center server 3 includes an E-mail transceiver 3.11, a text data generator 3.12, and an E-mail analyzer 3.13. The E-mail transceiver 3.11 exchanges electronic mails with the patient terminal 1 in the center server 3. The text data generator 3.12 converts the schedule data received from the schedule management unit 3.02 into a text data so that the schedule data is dispatched with an electronic mail. The E-mail analyzer 3.13 analyzes an electronic mail received at the E-mail transceiver 3.11, extracts the patient name, the result of activities according to the schedule data and the measured biodata, and registers the extracted data to the database 3.01.

### <3.2 Operation of the System>

The operation of the medical checkup network system of this embodiment will now be explained.

### (Step 1):

Similar to the first embodiment, the schedule data of each patient is entered by the medical staff who operate the doctor terminal 2.

### (Step 2):

The center server 3 transmits the schedule data stored in the patient data database 3.01 in the form of an electronic mail to the patient terminal 1.

In the center server 3, the schedule management unit 3.02 receives the current time from the time management unit 3.03, and compares the current time with the setting times for a plurality of schedules stored in the database 3.01. When the current time is identical to (or slightly earlier than) the setting time, details of the schedule data (for example, activities, patient name, mail address) corresponding to the setting time are transferred to the text data generator 3.12. The text data generator 3.12 converts the schedule data into a text data which can be transferred in an electronic mail, adds a mail address to the converted data, and transfers the data to the E-mail transceiver 3.11. The E-mail transceiver 3.11 incorporates a communication device such as a modem for transmitting the electronic mail to the patient terminal 1. It is noted that data carried in the electronic mail may include not only a text data but also audio and video data.

### (Step 3):

The patient terminal 1 receives the electronic mail from the center server 3 by the E-mail transceiver 1.13. Details of the electronic mail are notified to the patient in the form of messages, images, or sounds by the display 1.05 and the sound generator 1.11.

### (Step 4):

The result of medical activities determined by the schedule data is entered by the patient.

More specifically, the result of medical activities of the patient notified in Step 3 is entered into the response entry section 1.12. The response entry section 1.12 receives a response from the patient to transfer contents of response to the E-mail transceiver 1.13 in a form of a text data. The E-mail transceiver 1.13 returns the response data to predetermined addresses in the center server 3.

In the center server, The returned mail is received by the E-mail transceiver 3.11 and then transferred to the E-mail analyzer 3.13 by which the received mail is analyzed. The E-mail analyzer 3.13 extracts data indicating the contents of the response from the mail, and stores the extracted data in the database 3.01.

By conducting (Step 1) to (Step 4), the schedule data can be provided in the form of an electronic mail to the patient. Also, the electronic mail can be used for a return of the response from the patient which is entered at the response entry section 1.12. can be dispatched back as an electronic mail to the center server 3. The database 3.01 in the center server can store not only the schedule data of each patient but also the result of medical activity corresponding to the schedule of the patient. Using the patient terminal communication section 3.05 and the doctor terminal 2, the medical staff such as the doctor can access the data related to the patients.

While the electronic mail is dispatched from the center server to the patient terminal by a call from the center server in the above description, the patient terminal may call to the server to fetch the electronic mail.

The patient terminal 1 may comprise a mobile phone, a PHS, or a pager which can transmit and receive electronic mails. Since terminals including such a mobile phone are generally managed and used individually, the terminals befit to exchange personal data such as the schedule data.

It would be understood by those who skilled in the art that while the present invention is described in the form of the above embodiments, any other change, modification, or application can be made. The present invention is not limited to the foregoing disclosure but only defined by the appended claims.

## Claims

1. A medical checkup network system comprising:
a patient terminal for measuring a predetermined biodata of each patient such as a blood pressure or a body temperature;
a center server for storing the biodata measured by the patient terminal; and
a doctor terminal through which medical staff can view the biodata stored in the center server to conduct a diagnosis,
wherein the patient terminal, the doctor terminal, and the center server are connected with each other over a communication network.

2. A medical checkup network system comprising:
a doctor terminal for entering a predetermined medical support data such as an advice data or a schedule data directed to a patient;
a center server for storing the medical support data entered through the doctor terminal; and
a patient terminal for displaying the medical support data received from the center server,
wherein the patient terminal, the doctor terminal, and the center server are connected with each other over a communication network.

3. The medical checkup network system according to claim 1 or 2, wherein the center server has an authorizing section for providing the patient, the patient terminal, the medical staff or the doctor terminal registered in the center server with access right to enter a data or access the data stored in the center server.

4. The medical checkup network system according to claim 1 or 2, wherein the center server has an administrator terminal function for registering the user of the system and inputting the various medical data in the center server.

5. The medical checkup network system according to claim 1 or 2, wherein the center server is arranged for storing at least one software content to the patient terminal, the doctor terminal or the administrator terminal, and each of the terminals downloads the software content from the center server to use.

6. The medical checkup network system according to claim 5, wherein the software content of the patient terminal includes a version data indicative of a version of the-software content, and
the patient terminal compares the version data of the software content in the terminal with a latest version data managed in the center server upon communicating with the center server, and when the version data is older than the update version data, systematically downloads the latest version of the software content from the center server for version up.

7. The medical checkup network system according to claim 2, wherein
the center server stores the advice data directed to a patient entered at the doctor terminal,
the patient terminal has a section for detecting the reception of the advice data, and
the doctor terminal has a section for communicating with the center server and displaying whether or not the advice data is received by the patient terminal.

8. The medical checkup network system according to claim 4, wherein the administrator terminal registers, to the center server, an access right for the patient, the patient terminal, the doctor or the doctor terminal.

9. The medical checkup network system according to claim 4, wherein the administrator terminal enters patient terminal data which is data related to the patient terminal to be used by the patient.

10. The medical checkup network system according to claim 9, wherein the administrator terminal is arranged for executing at least one of procedures comprising: a procedure of entering identification number which identifies the patient terminal; a procedure of entering a name of a patient corresponding to the identification number; a procedure of entering identification code corresponding to the patient name; a procedure of entering at least one measurement item corresponding to the patient name; and a procedure of entering at least one name of instrument which senses biodata corresponding to the measurement item.

11. The medical checkup network system according to claim 1, wherein
the doctor terminal has a biodata threshold setting section for setting a threshold of the biodata for each patient, and
the center server has an alert section, the alert section receiving the threshold determined by the doctor terminal and providing the doctor terminal with an alert when the level of the biodata of the patient measured by the patient terminal exceeds the threshold.

12. The medical checkup network system according to claim 1, wherein
the doctor terminal has a sensitivity setting section for determining a level of sensitivity for receiving at the patient terminal a signal output from a sensor,
the center server has a section for receiving and storing the sensitivity level determined at the doctor terminal, and
the patient terminal has a section for communicating with the center server to receive the sensitivity level and modifying the sensitivity of the sensor based on the received sensitivity level.

13. The medical checkup network system according to claim 1 or 2, wherein the patient terminal has an initial connection setting section for communicating with the center server to execute a predetermined process upon being energized, and the initial connection setting section is arranged for performing at least one of automatically updating the software content, receiving the medical support data including the schedule data and the advice data, and transmitting measurement data which is not transferred.

14. The medical checkup network system according to claim 1, wherein
the patient terminal has a communicating section for measuring at least one kind of biodata to transmit the measured biodata to the center server,
the center server has a database for storing the biodata received from the patient terminal, and
the doctor terminal has a biodata displaying section for communicating with the center server and displaying the biodata stored in the database.

15. The medical checkup network system according to claim 14, wherein the patient terminal includes a measurement interface connected with at least one sensor for measuring the biodata, a biodata memory for storing the biodata measured by the sensor and received through the measurement interface, a communicating section for transmitting the biodata stored in the biodata memory and receiving the patient terminal data from the center server at the time of installation in the patient's home, and an instrument data memory for storing the identification number of each sensor to discriminate the sensor instruments from each other.

16. The medical checkup network system according to claim 15, wherein the patient terminal performs a procedure of connecting to the center server over the communication network at the time of installation, a procedure of receiving over the communication network from the center server patient terminal data which includes name of the patient corresponding to an identification number of the patient terminal, identification code corresponding to the patient name, measurement item corresponding to the patient name, instrument name of the sensor corresponding to the measurement item and control data of the health sensor, and a procedure of storing the patient terminal data.

17. The medical checkup network system according to claim 14, wherein the patient terminal includes a measurement interface connected with at least one sensor for measuring the biodata, a biodata memory section for storing the biodata measured by the sensor and received through the measurement interface, a communicating section for transmitting the biodata stored in the biodata memory section to the center server, an instrument data memory section for storing identification number to discriminate the sensor instruments from each other, and a recording medium interface for receiving the biodata from a detachable recording medium at the time of installation in the patient's home.

18. The medical checkup network system according to claim 17, wherein the patient terminal performs a procedure of receiving, at the time of installation in the patient's home, from a detachable recording medium, patient terminal data including at least one of name of the patient corresponding to identification number of the patient terminal, identification code corresponding to the patient name, measurement item corresponding to the patient name, instrument name of the health sensor corresponding to the measurement item, and control data of the sensor corresponding to the measurement item, and a procedure of storing the patient terminal data.

19. The medical checkup network system according to claim 2, comprising
the doctor terminal for receiving and monitoring a schedule data of the health care action for the patient,
the center server for storing the schedule data received from at least one doctor terminal, and
the patient terminal for communicating with the center server to provide the patient with the schedule data received from the center server.

20. The medical checkup network system according to claim 19, wherein the patient terminal has at least one of a displaying section for displaying the patient name, the setting time and the medical activities in the form of messages and images upon receiving the schedule data, and a sound generator for releasing a voice sound representing contents of the patient name, the setting time and the medical activities.

21. The medical checkup network system according to claim 19, wherein the schedule data includes at least one of pairs including a pair of the time and detail of dosage, a pair of the time of visit on the patient and name of a visitor or medical staff, a pair of the time of reservation and detail of the medical treatment at the medical facility, and a pair of the time and item of measurement of the biodata.

22. The medical checkup network system according to claim 19, wherein
the center server has a homepage builder for receiving the schedule data from the doctor terminal and converting the schedule data into data in an HTML or XML format, and a WEB server for storing the data related to the homepage, and
the patient terminal has a browser function for communicating with the center server, receiving the schedule data in the HTML or XML format, and displaying the schedule data.

23. The medical checkup network system according to claim 19, wherein
the center server has a mail transmitting section for storing the schedule data received from at least one doctor terminal and dispatching as an e-mail the medical activities to be done by the patient at the timing determined by the schedule data, and
the patient terminal has a receiving section for receiving the e-mail from the center server, and a displaying section for displaying details of the e-mail.

24. The medical checkup network system according to claim 19, wherein
the patient terminal has a response entering section for entering the result of the medical activities indicating whether or not the activities are performed according to the schedule data,
the center server has a database for communicating with the patient terminal, receiving the result of the medical activities from the patient terminal to store the result of activities, and
the doctor terminal has a section for communicating with the center server and receiving the result of the medical activities stored in the database to display the result.

25. The medical checkup network system according to claim 24, wherein
the response entering section in the patient terminal is implemented in an HTML or XML format over a browser, and
the center server has a WEB server for communicating with the browser in the patient terminal to receive the result of the medical activities, and a database for storing the result of the medical activities received at the WEB server section.

26. The medical checkup network system according to claim 24, wherein
the patient terminal has a mail transmitting section for converting the result of the medical activities into a text form data to transmit the converted data as an e-mail, and
the center server has an e-mail receiving section for receiving the e-mail from the patient terminal, an analyzing section for extracting a text data from the e-mail to check the result of the medical activities, and a database for storing the result of the medical activities.

27. The medical checkup network system according to claim 23 or 26, wherein the patient terminal comprises one of a mobile phone, a pager, and a PDA which can transmit and receive the e-mails.
